# EUROPÄISCHE PATENTSCHRIFT

(11) **EP 0 612 325 B1**
(45) Veröffentlichungstag und Bekanntmachung des Hinweises auf die Patenterteilung: **02.05.2003**
(21) Anmeldenummer: 92922212.3
(22) Anmeldetag: 28.10.1992
(51) Int. Cl.: C07K 14/36, C12N 15/31

(54) **REKOMBINANTES CORE-STREPTAVIDIN**
RECOMBINANT CORE-STREPTAVIDIN
STREPTAVIDINE A NOYAU DE RECOMBINAISON

(30) Priorität: 28.10.1991 DE 4135543
(43) Veröffentlichungstag der Anmeldung: 31.08.1994
(73) Patentinhaber: Roche Diagnostics GmbH, 68305 Mannheim (DE)
(72) Erfinder: KOPETZKI, Erhard, D-8122 Penzberg (DE); RUDOLPH, Rainer, D-8120 Weilheim (DE); GROSSMAN, Adelbert, D-8111 Eglfing (DE)
(74) Vertreter: Huber, Bernhard, Dipl.-Chem.
(86) Internationale Anmeldenummer: EP9202463
(87) Internationale Veröffentlichungsnummer: WO93009144

(56) Entgegenhaltungen:
- WO-A-86/02077
- WO-A-89/03422
- US-A- 4 839 293
- Proc. Natl. Acad. Sci., Band 87, Januar 1990, Takeshi Sano et al., "Expression of a cloned streptavidin gene in Escherichia coli"
- SANO T. AND CANTOR C.R.: 'Expression vectors for streptavidin-containing chimeric proteins' BIOCHEMICAL AND BIOPHYSICAL RESEARCH COMMUNICATIONS Bd. 176, Nr. 2, 1991, Seiten 571 - 577

## Beschreibung

Streptavidin ist natürlicherweise ein tetrameres Protein, wobei jede Untereinheit 159 Aminosäuren lang ist und ein Molekulargewicht von 16.450 Da aufweist. Die Gewinnung von Streptavidin erfolgt aus dem Kulturfiltrat von Streptomyces Avidinii (Chaiet, L. et al., Antimicrob. Agents Chemother. 3 (1963), 28-32). Streptavidin zeichnet sich, wie auch das aus dem Hühnereiweiß isolierte homologe Protein Avidin, durch eine außergewöhnlich starke nicht kovalente Bindung an das wasserlösliche Vitamin H (d-Biotin) aus. Es bindet ein Molekül Biotin pro Streptavidin-Untereinheit, also 4 Moleküle Biotin an das Tetramer. Die Dissoziationskonstante dieser Bindung beträgt 10⁻¹⁵ mol/l. Im Gegensatz zu Avidin ist das aus Streptomyces avidinii stammende Streptavidin nicht glykosyliert, enthält keine schwefelhaltigen Aminosäuren und besitzt einen niedrigeren isoelektrischen Punkt bei ca. pH 6,5. Das Avidin-Biotin-System, und insbesondere das Streptavidin-Biotin-System findet bereits eine weite Anwendung in der Diagnostik und Molekularbiologie (Wilchek, M. und Bayer, E.A., Methods Enzymol. 184 (1990), 5-13 und 14-45). Ein Beispiel für die Anwendung des Biotin-Streptavidin-Systems besteht darin, daß man Biotin oder Streptavidin an Target-Moleküle, z.B. Analyten, oder an eine Oberfläche, z.B. eine Reagenzgefäßoberfläche, chromatographische Medien oder Biopolymere, fixiert, wobei die Fixierung des Target-Moleküls z.B. dessen Nachweis ermöglicht.

Üblicherweise wird Streptavidin als sezerniertes Protein aus dem Kulturfiltrat von Streptomyces avidinii isoliert. Diese Streptavidin-Präparationen sind jedoch hinsichtlich der N-und/oder C-terminalen Aminosäuresequenz heterogen, was auf eine Proteolyse während der Fermentation (lange Fermentationszeiten) oder/und der Aufreinigung zurückzuführen ist (Argarana, C.E. et al., Nucl. Acids Res. 14 (1986) 1871-1882; Bayer, E.A. et al., Methods Enzymol. 184 (1990), 80-89; Pähler, A. et al., J. Biol. Chem. 262 (1987) 13933-13937; Hendrickson, W.A. et al., Proc. Natl. Acad. Sci. 86 (1989) 2190-2194; Bayer, E.A. et al., Biochem. J. 259 (1989) 369-376). Daneben neigt das vollständige native Streptavidin zur Aggregation (Pähler, A. et al., J. Biol. Chem. 262 (1987) 13933-13937; Bayer, E.A. et al., Biochem. J. 259 (1989) 369-376; Bayer, E.A. et al., J. Biochem. Biophys. Methods 13 (1986) 103-112). Folglich ist eine reproduzierbare Isolierung von nativen bzw. biologisch aktiven proteolytisch verkürzten Streptavidinproteinen aus S. avidinii problematisch.

Die bereits bei der Isolierung stattfindende Aggregation von nativem Streptavidin zu Oligomeren und seine geringe Löslichkeit (Pähler, A. et al., J. Biol. Chem. 262 (1987), 13933-13937) führt auch bei der Verwendung von Streptavidin im Streptavidin-Biotin-System zu Problemen, da ein nicht genau zu ermittelnder Anteil des Streptavidins der Reaktionsmischung entzogen ist, so daß Verfälschungen der Meßergebnisse möglich sind.

Ein weiterer Nachteil des nativen Streptavidins ist, daß es während der Fermentation und Aufarbeitung an seinem N- und C-Terminus proteolytisch limitiert prozessiert wird. So erhält man in der Regel ein Gemisch verschiedener Degradationsprodukte, wobei das Endprodukt dieser proteolytischen Prozessierung ein "Core"-Protein mit etwa 125 bis 127 Aminosäuren darstellt (Pähler, A. et al., J. Biol. Chem. 262 (1987) 13933-13937; Bayer, E.A. et al., Biochem. J. 259 (1989) 369-376). Die proteolytische Prozessierung verbessert das Bindeverhalten von Streptavidin zu Biotinkonjugaten (Bayer, E.A. et al., Biochem. J. 259 (1989) 369-376). Das üblicherweise erhaltene Gemisch von Degradationsprodukten zeigt jedoch kein genau reproduzierbares Bindeverhalten, so daß Verfälschungen der Meßergebnisse möglich sind.

Die rekombinante Herstellung von Streptavidin ist in der EP-B 0 198 015 beschrieben. Die heterologe Expression und Sekretion von nativem Streptavidin in E.coli mittels-der nativen Streptavidin-Signalsequenz führte zu Streptavidin-Varianten, die ins Periplasma sezerniert werden. Diese, als ECO-Avidine bezeichneten Streptavidin-Varianten sind jedoch ebenfalls im C-Terminus heterogen. Zudem wurde die S. avidinii-Signalsequenz in E.coli nicht vollständig abgespalten, wodurch der N-Terminus des Moleküls 13 zusätzliche Aminosäuren im Vergleich zu nativem Streptavidin enthält.

Sano und Cantor (Biochemical and Biophysical Research Communications, Vol. 176, No. 2, 1991) beschreiben ein rekombinant hergestelltes Protein, welches die Aminosäuren 16-133 des nativen Streptavidins enthält, allerdings C-terminal 8 zusätzliche Vektoraminosäuren trägt.

In einer Arbeit von Sano, T. und Cantor, C.R. (Proc. Natl. Acad. Sci. USA 87 (1990), 142-146) wird die heterologe Expression eines rekombinanten Streptavidins in E.coli, dessen Isolierung in Form von "Inclusion Bpdies" und die Renaturierung beschrieben. Dabei handelt es sich um ein N-terminal verkürztes Streptavidin-Fusionsprotein, welches die Aminosäuren 15 bis 159 des natürlichen Streptavidin-Moleküls enthält. Es zeigte sich jedoch, daß ein Teil (30 bis 50 %) des naturierten gereinigten Streptavidin-Fusionsproteins zu Oligomeren aggregiert.

Aufgabe der vorliegenden Erfindung war es, ein homogenes Streptavidin-Mutantenprotein (Core-Streptavidin) bereitzustellen, bei dem die Nachteile des Standes der Technik mindestens teilweise beseitigt sind. Ein weiteres Ziel der vorliegenden Erfindung war die Entwicklung eines sicheren, einfachen, reproduzierbaren und ökonomischen Herstellungsverfahrens für ein homogenes Core-Streptavidin, das für die verschiedenen analytischen und präparativen Anwendungen und insbesondere für diagnostische Tests geeignet ist.

Die erfindungsgemäße Aufgabe wird durch ein Verfahren zur Gewinnung von rekombinantem Core-Streptavidin gelöst, worin man Wirtszellen mit einer für ein Core-Streptavidin kodierenden DNA transformiert, eine Kultivierung der transformierten Wirtszellen unter geeigneten Bedingungen durchführt, eine Expression der für das Core-Streptavidin kodierenden DNA bewirkt und das rekombinante Core-Streptavidin aus den Wirtszellen oder dem Kulturmedium gewinnt, das dadurch gekennzeichnet ist, daß man eine für Core-Streptavidin kodierende DNA verwendet, welche
(a) die in SEQ ID NO. 1 gezeigte Nukleotidsequenz oder
(b) eine der Nukleotidsequenz (a) im Rahmen der Degeneration des genetischen Codes entsprechende Nukleotidsequenz aufweist.

Durch das erfindungsgemäße Verfahren gelingt es, ein Core-Streptavidin zu erhalten, welches homogen ist und daher in besonderem Maße für diagnostische Anwendungen geeignet ist. Das erfindungsgemäße Core-Streptavidin kann gegebenenfalls N-terminal einen Methioninrest enthalten.

Die Herstellung der für Core-Streptavidin kodierenden DNA, die Transformation geeigneter Wirtszellen und deren Kultivierung sowie die Expression und die Gewinnung des rekombinanten Produktes können nach den dem Fachmann geläufigen Methoden durchgeführt werden, wie sie typischerweise in "Molecular Cloning", T. Maniatis, I.F. Fritsch und J. Sambrook, Cold Spring Harbor Laboratory beschrieben sind. Die Herstellung der für core-Streptavidin kodierenden DNA kann ausgehend vom Streptavidin-Strukturgen (erhältlich z.B. von British Biotechnology limited) erfolgen, indem man das 3'-Ende um ein DNA-Fragment verkürzt, welches für die C-terminale Aminosäure-Sequenz IleAspAlaAlaLysLysAlaGlyValAsnAsnGlyAsnProLeuAspAlaValGlnGln kodiert und das 5'-Ende um ein DNA-Fragment verkürzt, das für die N-terminale Aminosäuresequenz AspProSerLysAspSerLysAlaGluValSerAla kodiert.

Zur direkten Expression (Initiation der Translation) von Core-Streptavidin muß an das 5'-Ende des Core-Streptavidin-Gens noch die für Met codierende Sequenz ATG angefügt werden. Das Methionin-Startcodon wird in Abhängigkeit von dem verwendeten Wirtsorganismus nach der Translation durch zelluläre Methionylaminopeptidase (Dalboge, H. et al., FEBS L. 266 (1990) 1-3) teilweise wieder abgespalten. Im Gegensatz zur Prozessierung des nativen Streptavidins bei der Sekretion und Aufreinigung aus Kulturfiltraten von S. avidinii beschränkt sich diese Prozessierung auf den N-terminalen Methioninrest und ist durch die Wahl der Bedingungen bei der Aufzucht des Mikroorganismus und der Expression in ihrem Ausmaß festgelegt, so daß entsprechend der erfindungsgemäßen Aufgabe ein reproduzierbar herstellbares Produkt von konstanter Zusammensetzung erhalten wird.

Die Auswahl des Expressionsvektors richtet sich nach der ausgewählten Wirtszelle. Für die verschiedenen Wirtszellen geeignete Vektoren sind dem Fachmann geläufig und brauchen hier nicht näher erläutert zu werden.

Als Wirtszelle wird zweckmäßig ein Mikroorganismus, z.B. ein Prokaryont oder Hefe verwendet. Besonders bevorzugt wird E.coli in Kombination mit einem für E.coli geeigneten Expressionsplasmid verwendet. Die Wirtszelle wird in an sich bekannter Weise mit einem rekombinanten Plasmid transformiert, das eine oder mehrere Kopien der erfindungsgemäß verwendeten Core-Streptavidin-kodierenden DNA enthält. Bevorzugt wird hierbei ein rekombinantes Expressionsplasmid eingesetzt, welches einen multi-copy-Replikationsursprung aufweist, d.h. in einer transformierten Zelle mit mindestens 20, vorzugsweise mit mindestens 100 Kopien pro Zelle vorliegt. Als multi-copy-Replikationsursprung eignet sich beispielsweise der Replikationsursprung des Plasmides pUC19.

Zur Herstellung des erfindungsgemäßen Core-Streptavidin wird die für das Core-Streptavidin-kodierende DNA in der transformierten Wirtszelle unter die Kontrolle eines gut regulierbaren Promotors gestellt. In diesem Fall ist vorzugsweise in der transformierten Wirtszelle ein zusätzliches Plasmid vorhanden, welches die Überexpression eines Repressorproteins bewirkt, das den regulierba-ren Promotor während der Anzuchtphase der Zellen inaktiviert. Bei dieser Ausführungsform des Verfahrens der Erfindung läßt sich dann die Expression der Core-Streptavidin-kodierenden DNA durch Zugabe eines den regulierbaren Promotor aktivierenden Induktors bewirken.

Die Expressionsbedingungen werden in an sich bekannter Weise vorzugsweise so eingestellt, daß das rekombinante Core-Streptavidin in Form der sogenannten "Inclusion Bodies" anfällt, da diese sich leicht von löslichen Zellbestandteilen abtrennen lassen. Die so gewonnenen "Inclusion Bodies" können dann ebenfalls in an sich bekannter Weise solubilisiert und zu löslichem biologisch aktivem Core-Streptavidin naturiert werden, beispielsweise nach dem in EP-A 253 823 beschriebenen Verfahren.

Das naturierte Core-Streptavidin wird dann zweckmäßig einer Feinreinigung unterworfen, vorzugsweise durch Chromatographie. Für die Chromatographie eignen sich beispielsweise Affinitätschromatographie-Materialien, Ionenaustauscher oder Materialien für die hydrophobe Chromatographie.

Ein weiterer Gegenstand der Erfindung ist eine rekombinante DNA, die für ein Core-Streptavidin kodiert und
(a) die in SEQ ID NO. 1 gezeigte Nukleotidsequenz oder
(b) eine der Nukleotidsequenz (a) im Rahmen der Degeneration des genetischen Codes entsprechende Nukleotidsequenz aufweist.

Ein weiterer Gegenstand der Erfindung ist ein rekombinanter Vektor, der mindestens eine Kopie der vorstehend definierten rekombinanten DNA enthält. Der Basisvektor ist zweckmäßig ein Plasmid, jedoch können auch virale Vektoren eingesetzt werden. Der Vektor der Erfindung besitzt vorzugsweise einen multi-copy-Replikationsursprung. Ferner befindet sich im erfindungsgemäßen Vektor die für Core-Streptavidin kodierende DNA vorzugsweise unter der Kontrolle eines regulierbaren Promotors.

Ein weiterer Gegenstand der Erfindung ist eine Wirtszelle, die mit der oben definierten rekombinanten DNA bzw. dem letztere enthaltenden Vektor transformiert ist.

Schließlich ist Gegenstand der Erfindung auch das rekombinante Core-Streptavidin, welches durch die in SEQ ID NO. 2 aufgeführte Aminosäuresequenz mit oder ohne N-terminalem Methioninrest gekennzeichnet ist.

Die in der vorliegenden Anmeldung genannten Plasmide und Mikroorganismen wurden bei der Deutschen Sammlung von Mikroorganismen und Zellkulturen GmbH (DSM), Mascheroder Weg 1B, D-3300 Braunschweig, gemäß den Bestimmungen des Budapester Vertrages unter den folgenden Hinterlegungsnummern hinterlegt:

| | |
|---|---|
| E. coli K12 RM 82 | DSM 5445 am 02.10.1991 |
| pSAM-core/pUBS 500 | DSM 6720 am 20.09.1991 |

In der Sequenzliste zeigt
SEQ ID NO. 1 die DNA-Sequenz von Core-Streptavidin,
SEQ ID NO. 2 die Proteinsequenz von Core-Streptavidin mit einem N-terminalen Methioninrest.
Fig. 1 zeigt die Plasmidkarte des Core-Streptavidin-Expressionsplasmids pSAM-Core.
Fig. 2 zeigt Circulardichroismus (CD)-Spektren von erfindungsgemäßem Core-Streptavidin (2a) bzw. von konventionellem Core-Streptavidin aus S. avidinii (2b) jeweils ohne (Kurve 1) und mit Biotin (Kurve 2).

Die folgenden Beispiele dienen zur weiteren Verdeutlichung der Erfindung.

### Beispiel 1

### 1.1 Konstruktion des C-terminal verkürzten Streptavidin-Gens (Plasmid pUC18-BBG9-C)

In der folgenden Plasmidkonstruktion wurde das 3'-Ende des nativen Streptavidin-Strukturgens um ein DNA-Fragment verkürzt, das für die C-terminale Aminosäuresequenz IleAspAlaAlaLysLysAlaGlyValAsnAsnGlyAsnProLeuAspAlaValGlnGln kodiert.

Hierzu wurde das Plasmid pUC18-BBG9 (pUC18 Vektor mit insertiertem Streptavidin-Gen, British Biotechnology Limited (WO 89/03422) mit den Restriktionsendonukleasen NheI und HindIII verdaut, das ca. 2,7 kBp lange NheI/HindIII-Fragment isoliert und mit dem synthetischen DNA-Fragment 
NheI PvuII
5'-CTAGTTAATGACAGCTGA-3'
3'-AATTACTGTCGACTTCGA-5'
AlaSer*** HindIII

***: Stopcodon
 ligiert (Konstruktion: pUC18-BBG9-C). Die gewünschte DNA-Sequenz wurde durch DNA-Sequenzierung bestätigt.

### 1.2 Konstruktion des E. coli Expressionsvektors pD-NX

Das Plasmid pD-NX ist ein Derivat des Plasmids pQE-6 (pDS56/RBSII,Ncol) der Firma Diagen (Düsseldorf), aus dem das promotorlose Chloramphenicol-Acetyltransferase Gen (CAT) entfernt wurde.

Dazu wurde das Plasmid pDS56/RBSII,Ncol mit den Restriktionsendonukleasen NheI und XbaI verdaut, das ca. 2,6 kBp lange NheI/XbaI-Vektorfragment isoliert und die kompatiblen Enden der NheI und XbaI Schnittstelle durch Ligation verknüpft (Konstruktion: pD-NX).

### 1.3 Konstruktion des N- und C-terminal verkürzten Streptavidin-Gens (Plasmid: pSA-core)

In der folgenden Plasmidkonstruktion wurde das 5'-Ende des nativen Streptavidin-Strukturgens um ein DNA-Fragment verkürzt, das für die N-terminale Aminosäuresequenz AspProSerLysAspSerLysAlaGlnValSerAla kodiert.

Hierzu wurde das wie oben beschrieben hergestellte Plasmid pUC18-BBG9-C mit der Restriktionsendonuklease PstI verdaut, das 3'-überhängende Ende der PstI-Schnittstelle mit T4-DNA-Polymerase abgedaut, die DNA mit HindIII nachgespalten, das ca. 390 Bp lange PstI(blunt)/ HindIII-Streptavidin-Fragment isoliert und in das ca. 2,6 kBp lange NcoI(blunt)/HindIII pD-NX Vektorfragment, nach Auffüllung des 5'-überhängenden Endes der NcoI-Schnittstelle mit Klenow-Polymerase, ligiert (Konstruktion: pSA-core).

### 1.4 Konstruktion des Core-Streptavidin "multicopy" Expressionsplasmids pSAM-core

Zur Erhöhung der Plasmidkopienzahl (Gendosiseffekt) in E. coli wurde der pMB1-Replikationsursprung des Plasmids pSA-core gegen den Replikationsursprung des "multicopy" Plasmids pUC19 ausgetauscht (Chambers, S.P. et al., Appl. Microbiol. Biotechnol. 29 (1988) 572-578).

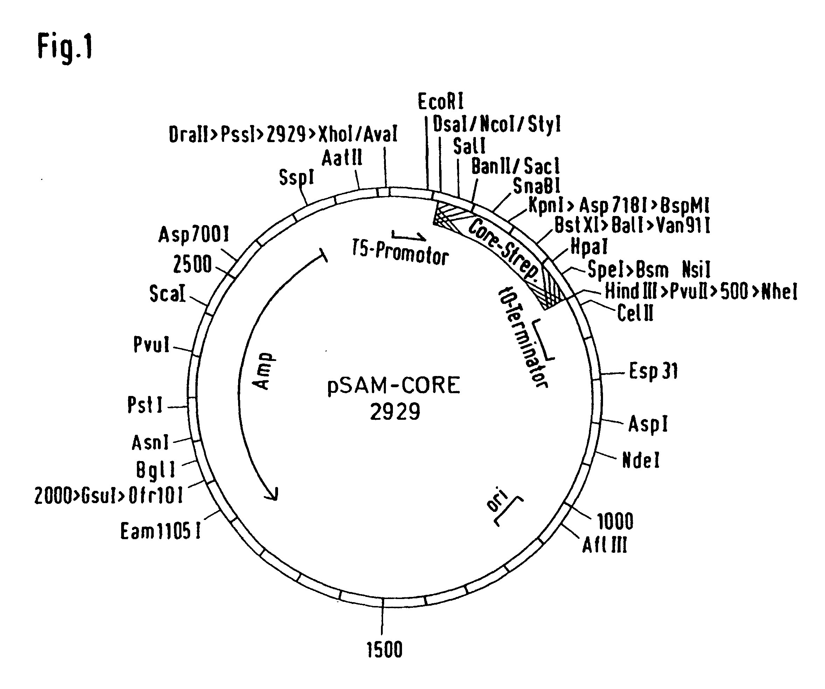

Hierzu wurde das ca. 1 kBp lange AflIII/BglI-Fragment aus pSA-core gegen das analoge Fragment aus pUC19 (Yanish-Perron, C. et al. Gene 33 (1985) 103-119) ausgetauscht (Konstruktion: pSAM-core). Die Plasmidkarte von pSAM core zeigt die Abb. 1. Das Plasmid pSAM-core ist als Plasmidgemisch mit pUBS500 unter DSM 6720 hinterlegt.

### Beispiel 2

### Expression von Core-Streptavidin in E. coli

Zur Expression von Core-Streptavidin wurde der E. coli K12-Stamm RM82 (DSM 5445) (eine Methionin-Revertante von ED 8654, Murray, N.E. et al., Mol. Gen. Genet. 150 (1977) 53-61) mit dem Core-Streptavidin Expressionsplasmid pSAM-core (Ampicillin-Resistenz) und dem lacI^{q}-Repressorplasmid pUBS500 (Kanamycin-Resistenz, Herstellung und Beschreibung siehe: EP-A 0 373 365) transformiert.

Die RM82/pUBS500/pSAM-core Zellen wurden in DYT-Medium (1% (w/v) Hefeextrakt, 1% (w/v) Bacto Tryptone, Difco, und 0,5% NaCl) mit 50 mg/l Ampicillin und 50 mg/l Kanamycin bis zu einer optischen Dichte bei 550 nm von 0,6 - 0,9 angezogen und anschließend mit IPTG (1 - 5 mmol/l Endkonzentration) induziert. Nach einer Induktionsphase von 4 - 8 Stunden wurden die Zellen durch Zentrifugation geerntet, mit 25 mmol/l Tris-HCl-Puffer, pH 7.5, gewaschen und bis zur Weiterverarbeitung bei -20°C gelagert.

### Beispiel 3

### Core-Streptavidin Expressionsanalyse in E. coli

Das Zellpellet aus 1 ml abzentrifugiertem Anzuchtmedium (RM82/pUBS500/pSAM-core Zellen) wurde in 0,25 ml 10 mmol/l Phosphatpuffer, pH 6.8, und 1 mmol/l EDTA resuspendiert und die Zellen durch Ultraschallbehandlung aufgeschlossen. Nach Zentrifugation wurde der Überstand mit 1/5 Volumen 5xSDS-Probenpuffer (1xSDS-Probenpuffer: 50 mmol/l Tris-HCl, pH 6.8, 1% SDS, 1% Mercaptoethanol, 10% Glycerin, 0.001% Bromphenolblau) versetzt. Die unlösliche Zelltrümmerfraktion wurde in 0,3 ml 1xSDS-Probenpuffer mit 6 - 8 M Harnstoff resuspendiert, die Proben 5 Minuten bei 95°C inkubiert und zentrifugiert. Danach wurden die Proteine durch SDS-Polyacrylamid Gelelektrophorese (PAGE) aufgetrennt (Laemmli, U.K., Nature 227 (1970) 680-685) und mit Coomassie Brilliant Blue R Farbstoff angefärbt.

Zudem wurden die elektrophoretisch aufgetrennten Proteine auf Nitrocellulosefilter transferiert, fixiert (Towbin, H. et al., Proc. Natl. Acad. Sci. 76 (1979) 4350) und die Streptavidin-immunreaktiven Proteine mit einem spezifischen Anti-Streptavidin-Antiserum aus Schaf detektiert.

Das in E. coli synthetisierte Core-Streptavidin Protein war homogen und wurde ausschließlich in der unlöslichen Zelltrümmerfraktion gefunden (IB's). Durch SDS-PAGE und Westernbiotanalyse konnten keine verkürzten Core-Streptavidin-Fragmente nachgewiesen werden. Die Expressionshöhe für Core-Streptavidin betrug 30 - 50 % bezogen auf das E.coli Gesamtprotein. Die Proteinsequenz des Core-Streptavidins ist in SEQ ID NO. 2 dargestellt.

### Beispiel 4

### Präparation von aktivem Core-Streptavidin aus E. coli

### 4.1 Zellyse und Präparation der "Inclusion bodies" (IB's)

40 g (Naßgewicht) E. coli RM82/pUBS500/pSAM-core Zellen wurden in 200 ml 0,1 mol/l Tris-HCl, pH 7.0, bei 0°C suspendiert, mit 60 mg Lysozym versetzt und 20 Minuten bei 0°C inkubiert. Nach Zugabe von 2 mmol/l MgCl₂ und 1 mg/100 ml DNase (Boehringer Mannheim GmbH, Kat.Nr. 104159) wurden die Zellen mechanisch mittels Hochdruckdispersion vollständig aufgeschlossen, danach wurde die DNA bei 25°C in 30 Minuten verdaut. Anschließend wurde die Aufschlußlösung mit 100 ml 60 mmol/l EDTA, 6% Triton X100 und 1,5 mol/l NaCl, pH 7.0 versetzt und weitere 30 Minuten bei 0°C inkubiert. Danach wurden die unlöslichen Bestandteile (Zelltrümmer und IB's) durch Zentrifugation mit einer Sorvall-Zentrifuge sedimentiert.

Das Pellet wurde in 200 ml 0,1 mol/l Tris-HCl, 20 mmol/l EDTA, pH 6.5 resuspendiert und 30 Minuten bei 25°C inkubiert; das IB-Präparat wurde dann durch Zentrifugation isoliert.

### 4.2 Solubilisierung der IB's

10 g IB-Pellet (Naßgewicht) wurden in 40 ml 0,1 mol/l Tris-HCl, 6 mol/l Guanidin-HCl, 10 mmol/l EDTA, pH 7.0 durch 1,5-stündiges Rühren bei 4°C suspendiert. Die unlöslichen Bestandteile wurden durch Zentrifugation abgetrennt und der klare Überstand wurde gegen 0,1 mol/l Tris-HCl, 6 mol/l Guanidin-HCl, 10 mmol/l EDTA, pH 7.0 (3 x 3 1, 24 Stunden, 4°C) dialysiert.

### 4.3 Naturierung

Die Renaturierung wurde bei 25°C durch 20fache Zugabe von jeweils 2 ml Core-Streptavidin Solubilisat (40 mg/ml) in 1,6 1 0,1 mol/l Natriumphosphat, 5 mmol/l EDTA, pH 7.0 im Zeitabstand von 20 Minuten bewirkt.

Nach Abschluß der Naturierungsreaktion wurden unlösliche Bestandteile durch Zentrifugation abgetrennt und der klare Core-Streptavidin-haltige Überstand weiterverarbeitet.

### 4.4 Konzentrierung und/oder Dialyse des Renaturierungsansatzes

Der Renaturierungsansatz kann bei Bedarf durch Membranfiltration konzentriert und/oder zur Entfernung von Guanidin-HCl gegen einen gewünschten Puffer dialysiert werden.

### Beispiel 5

### Reinigung von naturiertem Core-Streptavidin aus E. coli

Core-Streptavidin aus Renaturierungsansätzen kann bei Bedarf mit chromatographischen Methoden, die dem Fachmann bekannt sind, weiter gereinigt werden.

### Reinigung von Core-Streptavidin durch Ionenaustauschchromatographie an O-Sepharose ff nach vorheriger Konzentrierung und Dialyse

Der Renaturierungsansatz wurde in einer Rührzelle durch Ultrafiltration auf 120 ml konzentriert, zur Abtrennung von ungelösten Rückständen zentrifugiert und danach gegen 20 mmol/l Tris-HCl, pH 8.5, (2 x 10 1, 24 Stunden, 4°C) dialysiert. Eine mit dem gleichen Puffer äquilibrierte Q-Sepharose-ff-Säule (3 x 28 cm, V = 200 ml) wurde mit dem konzentrierten und dialysierten Renaturierungsansatz beladen (1 Säulenvolumen/Stunde, 1 SV/h) und so lange mit dem Äquilibrierungspuffer gewaschen, bis die Absorption des Eluats bei 280 nm den Leerwert des Puffers erreichte. Die Elution des gebundenen Materials erfolgte durch einen Gradienten von 0 bis 150 mmol/l NaCl in Äquilibrierungspuffer (10 SV, 1 SV/h).

| | Volumen | Aktivität¹⁾ | C_{Prot} | SA²⁾ |
|---|---|---|---|---|
| | ml | Einh./ml | mg/ml | Einh./mg |
| Dialysat | 180 | 34,8 | 2 | 17,4 |
| Q-Eluat | 200 | 24,8 | 1,3 | 19,4 |

| | | | | |
|---|---|---|---|---|
| 1) Aktivität im Titrationstest (vgl. Beispiel 6.2) | | | | |
| 2) Spezifische Aktivität: Aktivität im Titrationstest getellt durch Proteingehalt der Probe | | | | |

Geeignete Fraktionen aus der Elution wurden vereinigt (V = 200 ml), in der Rührzelle konzentriert, gegen bidest. Wasser dialysiert (2 x 15 1, 70 Stunden, 4°C), eingefroren und lyophilisiert.

### Beispiel 6

Charakterisierung von gereinigtem Core-Streptavidin aus E. coli

### 6.1 SDS-PAGE

Die Homogenität und Reinheit von naturiertem gereinigten Core-Streptavidin wurde durch SDS-PAGE (Laemmli, U.K., Nature 227 (1970) 680-685) untersucht.

Naturiertes gereinigtes Core-Streptavidin war homogen (einbandig) in der SDS-PAGE (Molekulargewicht: ca. 13 500 Da) mit einer Reinheit von > 98% bei ca. 70 - 90 %iger Prozessierung des N-terminalen Methionins.

### 6.2 Aktivitätsbestimmung (Biotin-Bindungstest)

Die Aktivität von Core-Streptavidin wurde durch Titration mit Biotin unter Verfolgung der durch die Komplexbildung verursachten Absorptionsänderung bei 233 nm bestimmt. 1 Einheit ist definiert als Bindung von 1 µg Biotin durch die Probe.

Die Proteinbestimmung erfolgte durch Messung der Extinktion bei 280 nm. Der Extinktionskoeffizient einer 1 %igen Lösung ist ∈ = 34 bei 280 mm (Green, M., Methods Enzymol. 184 (1990) 51-67).

Die gemessene spezifische Aktivität (19,4 Einheiten/mg Protein) entspricht einer Bindungsstöchiometrie Biotin : Core-Strept-avidin ≥ 1 (bezogen auf das Monomer).

### 6.3 Spektroskopische Charakterisierung

Die durch Biotin-Bindung verursachte Strukturänderung wurde mittels CD-Spektroskopie verfolgt. Hierzu wurden Spektren vor und nach Zugabe von Biotin registriert und diese mit ebenso erhaltenen Spektren von konventionell aus S. avidinii hergestelltem Core-Streptavidin verglichen.

Die Spektren wurden unter folgenden Bedingungen in einem Jasco J-600 CD-Spektrometer registriert: rec Core-Streptavidin bzw. Core-Streptavidin aus S. avidinii (Lyophilisat) wurde zu ca. 2 µmol/l in bidest. Wasser gelöst, in eine Küvette mit einem Lichtweg von 5 mm gegeben und mit einer Scan-Rate von 20 nm/Minute im Bereich von 200 bis 250 nm bei einer Dämpfung ("time const.") von 8 Sekunden gegen bidest. Wasser in der Referenzküvette vermessen. Anschließend wurde in die Meßküvette Biotin zu ca. 6 µmol/l zugesetzt und danach das Spektrum unter den gleichen Bedingungen erneut registriert.

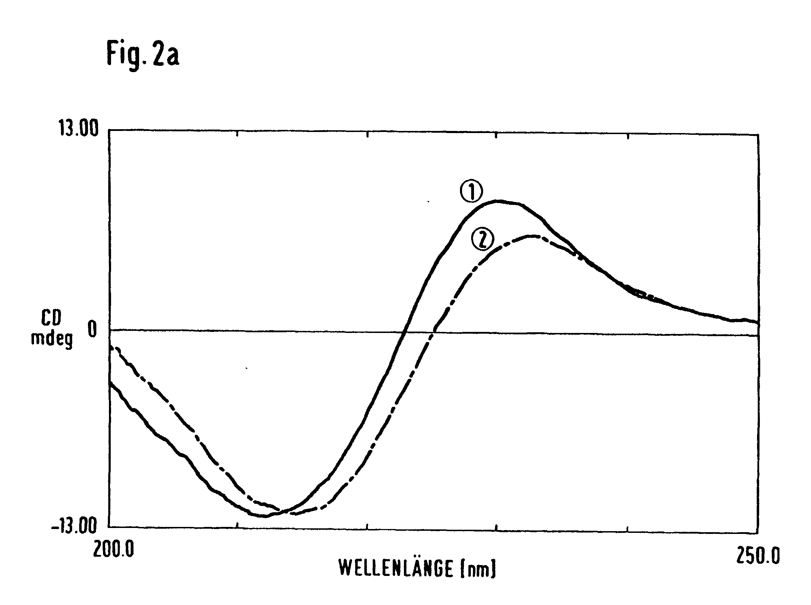

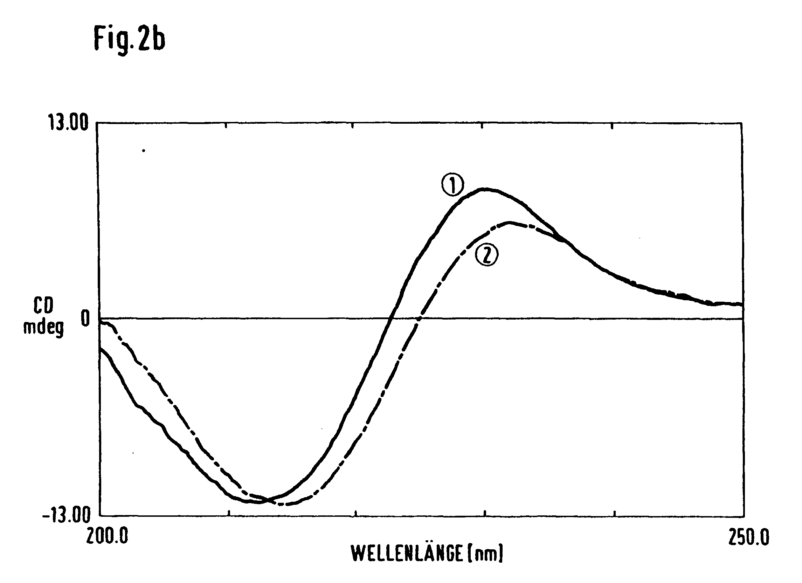

Spektren von gereinigtem Core-Streptavidin aus E. coli (Fig. 2a) und Spektren von konventionell hergestelltem Core-Streptavidin aus S. avidinii (Fig. 2b) sind nicht zu unterscheiden.

SEQ ID NO. 1

ART DER SEQUENZ: Nukleinsäure
SEQUENZLÄNGE: 387 Basenpaare

ATGGCCGAAG CTGGTATCAC TGGCACCTGG TATAACCAAC TGGGGTCGAC TTTCATTGTG 60

ACCGCTGGTG CTGACGGAGC TCTGACTGGC ACCTACGAAT CTGCGGTTGG TAACGCAGAA 120

TCCCGCTACG TACTGACTGG CCGTTATGAC TCTGCACCTG CCACCGATGG CTCTGGTACC 180

GCTCTGGGCT GGACTGTGGC TTGGAAAAAC AACTATCGTA ATGCGCACAG TGCCACTACG 240

TGGTCTGGCC AATACGTTGG CGGTGCTGAG GCTCGTATCA ACACTCAGTG GCTGTTAACA 300

TCCGGCACTA CCGAAGCGAA TGCATGGAAA TCGACACTAG TAGGTCATGA CACCTTTACC 360

AAAGTTAAGC CTTCTGCTGC TAGCTAA 387

SEQ ID NO. 2

ART DER SEQUENZ: Protein
SEQUENZLÄNGE: 128 Aminosäuren

Met Ala Glu Ala Gly Ile Thr Gly Thr Trp Tyr Asn Gln Leu Gly Ser 16

Thr Phe Ile Val Thr Ala Gly Ala Asp Gly Ala Leu Thr Gly Thr Tyr 32

Glu Ser Ala Val Gly Asn Ala Glu Ser Arg Tyr Val Leu Thr Gly Arg 48

Tyr Asp Ser Ala Pro Ala Thr Asp Gly Ser Gly Thr Ala Leu Gly Trp 64

Thr Val Ala Trp Lys Asn Asn Tyr Arg Asn Ala His Ser Ala Thr Thr 80

Trp Ser Gly Gln Tyr Val Gly Gly Ala Glu Ala Arg Ile Asn Thr Gln 96

Trp Leu Leu Thr Ser Gly Thr Thr Glu Ala Asn Ala Trp Lys Ser Thr 112

Leu Val Gly His Asp Thr Phe Thr Lys Val Lys Pro Ser Ala Ala Ser 128

## Patentansprüche

1. Verfahren zur Gewinnung von rekombinantem Core-Streptavidin, worin man Wirtszellen mit einer für ein Core-Streptavidin kodierenden DNA transformiert, eine Kultivierung der transformierten Wirtszellen unter geeigneten Bedingungen durchführt, eine Expression der für das Core-Streptavidin kodierenden DNA bewirkt und das rekombinante Core-Streptavidin aus den Wirtszellen oder dem Kulturmedium gewinnt,
**dadurch gekennzeichnet,**
**daß** man eine für Core-Streptavidin kodierende DNA verwendet, welche
(a) die in SEQ ID NO. 1 gezeigte Nukleotidsequenz oder
(b) eine der Nukleotidsequenz (a) im Rahmen der Degeneration des genetischen Codes entsprechende Nukleotidsequenz aufweist.

2. Verfahren nach Anspruch 1,
**dadurch gekennzeichnet, daß** man eine mikrobielle Wirtszelle verwendet.

3. Verfahren nach Anspruch 2,
**dadurch gekennzeichnet, daß** man eine prokaryontische Wirtszelle verwendet.

4. Verfahren nach Anspruch 3,
**dadurch gekennzeichnet, daß** man E.coli als Wirtszelle verwendet.

5. Verfahren nach Anspruch 1,
**dadurch gekennzeichnet, daß** man eine eukaryontische Wirtszelle verwendet.

6. Verfahren nach Anspruch 5,
**dadurch gekennzeichnet, daß** man Hefe als Wirtszelle verwendet.

7. Verfahren nach einem der Ansprüche 1 bis 6,
**dadurch gekennzeichnet, daß** man die Wirtszelle mit einem rekombinanten Plasmid transformiert, das mindestens eine Kopie der für das Core-Streptavidin kodierenden DNA enthält.

8. Verfahren nach Anspruch 7,
**dadurch gekennzeichnet, daß** man ein rekombinantes Plasmid mit einem "multi copy" Replikationsursprung verwendet.

9. Verfahren nach einem der vorhergehenden Ansprüche,
**dadurch gekennzeichnet, daß** sich die für das Core-Streptavidin kodierende DNA in der transformierten Wirtszelle unter Kontrolle eines regulierbaren Promotors befindet.

10. Verfahren nach Anspruch 9,
**dadurch gekennzeichnet, daß** man die Expression der für das Core-Streptavidin kodierenden DNA durch Zugabe eines Induktors für den regulierbaren Promotor bewirkt.

11. Verfahren nach einem der vorhergehenden Ansprüche,
**dadurch gekennzeichnet, daß** man das rekombinante Core-Streptavidin aus den Wirtszellen in Form von Inclusion Bodies gewinnt und anschließend eine Solubilisierung und Naturierung der Inclusion Bodies zu löslichem, biologisch aktivem Core-Streptavidin durchführt.

12. Verfahren nach Anspruch 11,
**dadurch gekennzeichnet, daß** man das naturierte Core-Streptavidin chromatographisch reinigt.

13. Rekombinante DNA,
**dadurch gekennzeichnet, daß** sie für ein Core-Streptavidin kodiert und
(a) die in SEQ ID NO. 1 gezeigte Nukleotidsequenz oder
(b) eine der Nukleotidsequenz (a) im Rahmen der Degeneration des genetischen Codes entsprechende Nukleotidsequenz aufweist.

14. Rekombinanter Vektor,
**dadurch gekennzeichnet, daß** er mindestens eine Kopie einer DNA nach Anspruch 13 enthält.

15. Vektor nach Anspruch 14,
**dadurch gekennzeichnet, daß** er ein Plasmid ist.

16. Vektor nach Anspruch 15,
**dadurch gekennzeichnet, daß** er einen "multi copy" Replikationsursprung besitzt.

17. Vektor nach einem der Ansprüche 14 bis 16,
**dadurch gekennzeichnet, daß** sich die für das Core-Streptavidin kodierende DNA unter Kontrolle eines regulierbaren Promotors befindet.

18. Plasmid pSAM-core DSM 6720

19. Wirtszelle,
**dadurch gekennzeichnet, daß** sie mit einer DNA nach Anspruch 13 oder einem Vektor nach einem der Ansprüche 14 bis 18 transformiert ist.

20. Rekombinantes Core-Streptavidin,
**dadurch gekennzeichnet, daß** es
(a) die in SEQ ID NO. 2 gezeigte Aminosäuresequenz und/oder
(b) die in SEQ ID NO. 2 gezeigte Aminosäuresequenz ohne den N-terminalen Met-Rest
aufweist.

21. Verwendung eines rekombinanten Core-Streptavidins nach Anspruch 20 für diagnostischer in vitro Testverfahren und Affinitätschromatographie.

## Claims

1. Method for the isolation of recombinant core streptavidin wherein host cells are transformed with a DNA coding for a core streptavidin, the transformed host cells are cultured under suitable conditions, the DNA coding for the core streptavidin is expressed and the recombinant core streptavidin is isolated from the host cells or from the culture medium, wherein a DNA coding for core streptavidin is used comprising:
(a) the nucleotide sequence shown in SEQ ID NO. 1, or
(b) a nucleotide sequence corresponding to a nucleotide sequence (a) falling within the scope of the degeneracy of the genetic code.

2. Method as claimed in claim 1, wherein a microbial host cell is used.

3. Method as claimed in claim 2, wherein a prokaryotic host cell is used.

4. Method as claimed in claim 3, wherein E. coli is used as a host cell.

5. Method as claimed in claim 1, wherein a eukaryotic host cell is used.

6. Method as claimed in claim 5, wherein yeast is used as a host cell.

7. Method as claimed in one of the claims 1 to 6, wherein the host cell is transformed with a recombinant plasmid which contains at least one copy of the DNA coding for core streptavidin.

8. Method as claimed in claim 7, wherein a recombinant plasmid with a multicopy replication origin is used.

9. Method as claimed in any of the preceding claims, wherein the DNA coding for core streptavidin in the transformed host cell is placed under the control of a regulatory promoter.

10. Method as claimed in claim 9, wherein the DNA coding for the core streptavidin is expressed by addition of an inductor for the regulatory promoter.

11. Method as claimed in one of the preceding claims, wherein the recombinant core streptavidin is isolated from the host cells in the form of inclusion bodies which are subsequently solubilized and reassembled to form soluble, biologically-active core streptavidin.

12. Method as claimed in claim 11, wherein the reassembled core streptavidin is purified by chromatography.

13. Recombinant DNA, wherein it codes for a core streptavidin and comprises
(a) the nucleotide sequence shown in SEQ, ID, No. 1 or
(b) a nucleotide sequence corresponding to a nucleotide sequence (a) falling within the scope of the degeneracy of the genetic code.

14. Recombinant vector, wherein the vector contains at least one copy of a DNA as claimed in claim 13.

15. Vector as claimed in claim 14, wherein the vector is a plasmid.

16. Vector as claimed in claim 15, wherein the vector has a multicopy origin of replication.

17. Vector as claimed in one of the claims 14 to 16, wherein the DNA coding for the core streptavidin is under the control of a regulatory promoter.

18. Plasmid pSAM-core DSM 6720.

19. A host cell wherein the host cell is transformed with a DNA as claimed in claim 13 or with a vector as claimed in any of the claims 14 to 18.

20. Recombinant core streptavidin, wherein the recombinant core streptavidin comprises
(a) the amino acid sequence shown in SEQ ID No. 2 and /or
(b) the amino acid sequence shown in SEQ ID No. 2 without the N-terminal Met residue.

21. Use of a recombinant core streptavidin as claimed in claim 20 for diagnostic in vitro test procedures and affinity chromatography.

## Revendications

1. Procédé d'obtention de streptavidine-coeur (core-streptavidine) recombinante, où l'on transforme des cellules hôtes avec un ADN codant une streptavidine-coeur (core-streptavidine), réalise une culture des cellules hôtes transformées dans des conditions appropriées, déclenche une expression de l'ADN codant la streptavidine-coeur (core-streptavidine) et obtient la streptavidine-coeur (core-streptavidine) recombinante à partir des cellules hôtes ou du milieu de culture,
**caractérisé en ce que**
l'on utilise un ADN codant la streptavidine-coeur (core-streptavidine) qui présente
(a) la séquence de nucléotide montrée dans la SEQ ID N°1 ou
(b) une séquence de nucléotides correspondant à la séquence de nucléotides (a) en tenant compte de la dégénérescence du code génétique.

2. Procédé selon la revendication 1,
**caractérisé en ce que**
l'on utilise une cellule hôte microbienne.

3. Procédé selon la revendication 2,
**caractérisé en ce que**
l'on utilise une cellule hôte procaryote.

4. Procédé selon la revendication 3,
**caractérisé en ce que**
l'on utilise E. coli comme cellule hôte.

5. Procédé selon la revendication 1,
**caractérisé en ce que**
l'on utilise une cellule hôte eucaryote.

6. Procédé selon la revendication 5,
**caractérisé en ce que**
l'on utilise la levure comme cellule hôte.

7. Procédé selon l'une des revendications 1 à 6,
**caractérisé en ce que**
l'on transforme la cellule hôte avec un plasmide recombinant qui contient au moins une copie de l'ADN codant la streptavidine-coeur (core-streptavidine).

8. Procédé selon la revendication 7,
**caractérisé en ce que**
l'on utilise un plasmide recombinant avec une origine de réplication multicopie.

9. Procédé selon l'une des revendications précédentes,
**caractérisé en ce que**
l'ADN codant la streptavidine-coeur (core-streptavidine) dans la cellule transformée se trouve sous le contrôle d'un promoteur régulable.

10. Procédé selon la revendication 9,
**caractérisé en ce que**
l'on provoque l'expression de l'ADN codant l'expression de l'ADN codant la streptavidine-coeur (core-streptavidine) par addition d'un inducteur pour le promoteur régulable.

11. Procédé selon l'une des revendications précédentes,
**caractérisé en ce que**
l'on obtient la streptavidine-coeur (core-streptavidine) recombinante à partir de cellules hôtes sous forme de corps d'inclusion et réalise ensuite une solubilisation et naturation des corps d'inclusion en streptavidine-coeur (core-streptavidine) soluble, biologiquement active.

12. Procédé selon la revendication 11,
**caractérisé en ce que**
l'on purifie par chromatographie la streptavidine-coeur (core-streptavidine) naturée.

13. ADN recombinant,
**caractérisé en ce qu'**
il code une streptavidine-coeur (core-streptavidine) et qu'il présente
(a) la séquence de nucléotide montrée dans la SEQ ID N°1 ou
(b) une séquence de nucléotides correspondant à la séquence de nucléotides (a) en tenant compte de la dégénérescence du code génétique.

14. Vecteur recombinant,
**caractérisé en ce qu'**
il contient au moins une copie d'un ADN selon la revendication 13.

15. Vecteur selon la revendication 14,
**caractérisé en ce qu'**
il est un plasmide.

16. Vecteur selon la revendication 15,
**caractérisé en ce qu'**
il possède une origine de réplication multicopie.

17. Vecteur selon l'une des revendications 14 à 16, **caractérisé en ce que**
l'ADN codant la streptavidine-coeur (core-streptavidine) se trouve sous le contrôle d'un promoteur régulable.

18. Plasmide pSAM-core DSM 6720

19. Cellule hôte,
**caractérisée en ce qu'**
elle est transformée avec un ADN selon la revendication 13 ou un vecteur selon l'une des revendications 14 à 18.

20. Streptavidine-coeur (core-streptavidine) recombinante
**caractérisée en ce qu'**
elle présente
(a) la séquence d'acides aminés montrée dans la SEQ ID N° 2 et/ou
(b) la séquence d'acides aminés montrée dans SEQ ID N° 2 sans le résidu Met N-terminal.

21. Utilisation d'une streptavidine-coeur (core-streptavidine) recombinante selon la revendication 20 pour les procédés de test diagnostic in vitro et chromatographie d'affinité.
